# EUROPEAN PATENT SPECIFICATION

(11) **EP 3 111 946 B1**
(45) Date of publication and mention of the grant of the patent: **19.08.2020**
(21) Application number: 16176595.3
(22) Date of filing: 28.06.2016
(51) Int. Cl.: A61K 35/16, A61K 35/28, A61P 17/02

(54) **WOUND HEALING COMPOSITION**
ZUSAMMENSETZUNG FÜR WUNDHEILUNG
COMPOSITION DE CICATRISATION

(30) Priority: 30.06.2015 BE 201505411
(43) Date of publication of application: 04.01.2017
(73) Proprietor: Wouters, Guido, 2580 Putte (BE)
(72) Inventor: Wouters, Guido, 2580 Putte (BE)
(74) Representative: Pappaert, Kris

(56) References cited:
- WO-A1-2013/045689
- PUKANA JAYARAMAN ET AL: "Stem cells conditioned medium: a new approach to skin wound healing management", CELL BIOLOGY INTERNATIONAL., vol. 37, no. 10, 24 June 2013 (2013-06-24) , pages 1122-1128, XP055228896, GB ISSN: 1065-6995, DOI: 10.1002/cbin.10138
- BHANG SUK HO ET AL: "Platelet-Rich Plasma Enhances the Dermal Regeneration Efficacy of Human Adipose-Derived Stromal Cells Administered to Skin Wounds", CELL TRANSPLANTATION, vol. 22, no. 3, 2013, pages 437-445, XP002751123,
- MARIA G. ROUBELAKIS ET AL: "Platelet-Rich Plasma (PRP) Promotes Fetal Mesenchymal Stem/Stromal Cell Migration and Wound Healing Process", STEM CELL REVIEWS, vol. 10, no. 3, 6 February 2014 (2014-02-06), pages 417-428, XP055229659, US ISSN: 1550-8943, DOI: 10.1007/s12015-013-9494-8
- PARK BYUNG-SOON ET AL: "Hair growth stimulated by conditioned medium of adipose-derived stem cells is enhanced by hypoxia: evidence of increased growth factor secretion", BIOMEDICAL RESEARCH, BIOMEDICAL RESEARCH PRESS INC., SAPPORO, JP, vol. 31, no. 1, 1 February 2010 (2010-02-01), pages 27-34, XP009158891, ISSN: 0388-6107
- ZHENG JUN LI ET AL: "Autologous Platelet-Rich Plasma: A Potential Therapeutic Tool for Promoting Hair Growth", DERMATOLOGIC SURGERY., vol. 38, no. 7 part 1, 1 July 2012 (2012-07-01), pages 1040-1046, XP055202490, NEW YORK, US ISSN: 1076-0512, DOI: 10.1111/j.1524-4725.2012.02394.x
- PHUC VAN PHAM ET AL: "Activated platelet-rich plasma improves adipose-derived stem cell transplantation efficiency in injured articular cartilage", STEM CELL RESEARCH & THERAPY, BIOMED CENTRAL LTD, LONDON, UK, vol. 4, no. 4, 1 August 2013 (2013-08-01), page 91, XP021162896, ISSN: 1757-6512, DOI: 10.1186/SCRT277
- Allan Mishra ET AL: "Buffered Platelet-Rich Plasma Enhances Mesenchymal Stem Cell Proliferation and Chondrogenic Differentiation", Tissue Engineering Part C: Methods, vol. 15, no. 3, 1 September 2009 (2009-09-01), pages 431-435, XP055139554, ISSN: 1937-3384, DOI: 10.1089/ten.tec.2008.0534

## Description

### FIELD OF THE INVENTION

The present invention relates to compositions for use in pharmaceutical, cosmetic and cosmeceutical applications, particularly wound healing, including the treatment of lesions and burns. In particular, the present invention relates to compositions comprising platelet enriched plasma (PRP) and stem cell conditioned medium (SC CM) for use in pharmaceutical, cosmetic and cosmeceutical applications such as wound healing.

### BACKGROUND OF THE INVENTION

Wound healing is a process which passes through several phases. Initially, in the inflammatory phase the body responds to the injury by contracting the blood vessels in the wound bed and the formation of a clot. Once haemostasis has been achieved, blood vessels then dilate to allow essential cells; antibodies, white blood cells, growth factors, enzymes and nutrients to reach the wounded area. This leads to a rise in exudate levels so the surrounding skin needs to be monitored for signs of maceration. It is at this stage that the characteristic signs of inflammation can be seen; erythema, heat, oedema, pain and functional disturbance. The predominant cells at work here are the phagocytic cells; 'neutrophils and macrophages'; mounting a host response and autolysing any devitalised 'necrotic / sloughy' tissue.

During proliferation, the wound is 'rebuilt' with new granulation tissue which is comprised of collagen and extracellular matrix and into which a new network of blood vessels develop, a process known as 'angiogenesis'. Healthy granulation tissue is dependent upon the fibroblast receiving sufficient levels of oxygen and nutrients supplied by the blood vessels. Healthy granulation tissue is granular and uneven in texture; it does not bleed easily and is pink/red in colour. The colour and condition of the granulation tissue is often an indicator of how the wound is healing. Dark granulation tissue can be indicative of poor perfusion, ischaemia and/or infection. Epithelial cells finally resurface the wound, a process known as 'epithelialisation'. Maturation is the final phase and occurs once the wound has closed. This phase involves remodelling of collagen from type III to type I. Cellular activity reduces and the number of blood vessels in the wounded area regress and decrease.

Stem cells are of great interest in numerous therapeutic, cosmetic and cosmeceutical areas because of their capacity to form cells of multiple types. The culture media used to grow cells, including stem cells, have been described for therapeutic, cosmetic and cosmeceutical uses arising from the secretion by the growing cells of proteins and other factors into the media. See for example US7,118.746; US7,160,726 and WO2008/020815.

Also, platelet enriched plasma (PRP), a product of blood plasma that is rich in platelets, is known for use in a variety of therapeutic or cosmetic applications including enhancing wound healing in dental implants and sinus elevations, heart surgery, orthopaedic surgery and dermatology (chronic wound healing). For instance WO2005/065269 discloses several compositions comprising PRP and fibroblast cells for the treatment of skin, in particular, repeated administration of PRP in a dermatologically acceptable carrier to skin to e.g. reduce appearance of wrinkles. Despite the above, there remains a need for alternative compositions for therapeutic, cosmetic and cosmeceutical purposes. It is particularly desirable to identify new types of compositions which work more efficiently compared to the already existing products.

### SUMMARY OF THE INVENTION

In a first aspect, the present invention relates to a composition comprising platelet enriched plasma (PRP) and stem cell conditioned medium (SC CM), wherein said SC CM is the medium harvested after the culturing of mesenchymal stem cells.

More particular, in the composition according to the present invention said PRP comprises transforming growth factor-β (TGF-β), fibrinogen, platelet-derived growth factor (PDGF), epidermal growth factor (EGF), transforming growth factor-α (TGF-α), vascular endothelial growth factor (VEGF), platelet thrombo-plastin, thrombospondin, coagulation factors, calcium, serotonin, histamine, and hydrolytic enzymes.

More particular, in the composition according to the present invention said SC CM comprises hepatocyte growth factor, transforming growth factor β (TGF-β), anti-apoptopic factors, keratinocyte growth factor, brain-derived neurotrophic factor (BDNF), Flt-3 ligand, granulocyte colony stimulating factor (G-CSF), granulocyte/macrophage colony stimulating factor (GM-CSF), macrophage colony stimulating factor (M-CSF), interleukin-6 (IL-6), interleukin-7 (IL-7), interleukin-8 (IL-8), interleukin-11 (IL-11), interleukin -12 (IL-12), leukemia inhibitory factor (LIF), and tumor necrosis factor-alpha (TNF-α).

In particular, the composition according to the present invention further comprises DMSO.

In particular, the composition according to the present invention further comprises a gelling agent.

More particular, in the composition according to the present invention said PRP and SC CM are present in the composition in a ratio of about 3:1 to about 1:3.

In particular, in the composition according to the present invention said SC CM is prepared from stem cells isolated from bone marrow or adipose tissue.

More particular, in the composition according to the present invention said conditioned medium is a processed conditioned medium.

According to a second aspect, the present invention relates to the compositions as described herein for use in (animal) medicine, more in particular for use in the treatment of skin wounds in a subject and/or mastitis. said subject is a mammal, preferably selected from domestic animals such as dogs, cats and rabbits or farm animals such as horses, cattle, sheep and goats.

Disclosed is the use of the compositions as described herein for promoting hair growth in a mammalian subject.

According to a third aspect, the present invention relates to a method for preparing a composition according to the present invention, comprising at least the blending of platelet enriched plasma (PRP) and stem cell conditioned medium (SC CM), wherein said SC CM medium is harvested after the culturing of mesenchymal stem cells.

### BRIEF DESCRIPTION OF FIGURES

**Figure 1** shows the effect of the composition of the invention on wounds in hedgehogs.
**Figure 2** shows the effect of the composition of the invention on wounds in hedgehogs.
**Figure 3** shows the effect of the composition of the invention on wounds in horses.
**Figure 4** shows the effect of the composition of the invention on wounds in dogs.
**Figure 5** shows the effect of the composition of the invention on wounds in mice.
**Figure 6** shows the effect of the composition of the invention on wounds in elephants.
Figure 7 is a representation of the wound closure. Wound area is calculated from the average of three daily diameter measurements along the x, y and z-axes. Wound closure is expressed as a percentage of initial wound area at day 0.

### DETAILED DESCRIPTION OF THE INVENTION

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

The present invention provides in compositions comprising both platelet enriched plasma (PRP) and stem cell conditioned medium (SC CM), wherein said SC CM is the medium harvested after the culturing of mesenchymal stem cells.

The term "PRP" or "platelet rich plasma" as used herein should be understood to mean a blood product which comprises platelets concentrate in a small volume of plasma. Platelet enriched plasma (PRP) is obtained by processing blood to obtain platelet enriched plasma for instance by double centrifugation designed to separate the PRP aliquot from platelet-poor plasma and red blood cells.

The term "SC CM" or "stem cell conditioned medium" as used herein should be understood to mean a medium that has been harvested from cultured mesenchymal stem cells. Stem cell conditioned medium typically contains metabolites, growth factors, and extracellular matrix proteins secreted into the medium by the cultured stem cells. Examples of each might include: metabolites such as glucose, amino acids, and nucleosides; growth factors such as interleukins, EGF (epidermal growth factor), and PDGF (platelet-derived growth factor); and matrix proteins such as collagen, fibronectin, and various proteoglycans.

While therapeutic treatments with platelet rich plasma and stem cell conditioned medium separately have been shown great promises, it has now been found that the combination of both creates a synergistic effect. In particular for the healing of wounds, typical healing times with the products on the market range between 3 to 6 weeks, while the combination of platelet enriched plasma and stem cell conditioned medium allows a further reduction of the healing time to 1 to 2 weeks.

It is important in the healing of surgical or traumatic wounds that the healing occurs quick and without delays, in the interest of the costs as well as the success of the healing process. The composition according to the invention has been found to provide in a successful and fast cutaneous wound healing process. A rich source of the complex group of growth factors in the product allows the natural repair of the wound. The platelets act in the haemostasis; wound healing and re-epithelialization liberating diverse GF's that stimulate the angiogenesis, promoting growth and vascular fibroblast proliferation that in turn provide an increase in the collagen synthesis. Both Platelet-rich plasma (PRP) and stem cell conditioned medium (SC CM) is 100% biocompatible and safe. The healing rate and the regaining of the original skin reveals the high regenerative potential.

Accordingly, in a first aspect, the present invention therefore provides in compositions comprising platelet enriched plasma (PRP) and stem cell conditioned medium (SC CM), wherein said SC CM is the medium harvested after culturing of mesenchymal stem cells.

Preferably, in the compositions according to the invention said PRP comprises at least transforming growth factor-β (TGF-β), fibrinogen, platelet-derived growth factor (PDGF), epidermal growth factor (EGF), transforming growth factor-α (TGF-α), vascular endothelial growth factor (VEGF), platelet thrombo-plastin, thrombospondin, coagulation factors, calcium, serotonin, histamine, and hydrolytic enzymes.

To the PRP fraction also other substances may be added including for instance antioxidants such as vitamins such as vitamin C (ascorbic acid), vitamin E, vitamin A and other retinoids; and the carotenes such as beta.-carotene. A synergetic effect was observed when the cells from which the conditioned medium is harvested were mesenchymal stem cells. Mesenchymal stem cells (MSCs) are multipotent stromal cells that can differentiate into a variety of cell types, including: osteoblasts (bone cells), chondrocytes (cartilage cells), myocytes (muscle cells) and adipocytes (fat cells). Typically, MSCs are harvested from bone marrow, adipose tissue, the placenta, the umbilical cord blood, adult muscle, corneal stroma or the dental pulp of deciduous baby teeth and cultured for a certain period in time (typically 2 weeks) before harvesting the cells and the conditioned medium. The conditioned medium is rich in growth factors such as hepatocyte growth factor, transforming growth factor β, anti-apoptopic factors, keratinocyte growth factor, brain-derived neurotrophic factor (BDNF), Flt-3 ligand, granulocyte colony stimulating factor (G-CSF), granulocyte/macrophage colony stimulating factor (GM-CSF), macrophage colony stimulating factor (M-CSF), interleukin-6 (IL-6), interleukin-7 (IL-7), interleukin-8 (IL-8), interleukin-11 (IL-11), interleukin-12 (IL-12), leukemia inhibitory factor (LIF), and tumor necrosis factor-alpha (TNF-α). Accordingly, in the composition according to the present invention said SC CM comprises at least hepatocyte growth factor, transforming growth factor β (TGF-β), anti -apoptopic factors, keratinocyte growth factor, brain-derived neurotrophic factor (BDNF), Flt-3 ligand, granulocyte colony stimulating factor (G-CSF), granulocyte/macrophage colony stimulating factor (GM-CSF), macrophage colony stimulating factor (M-CSF), interleukin-6 (IL-6), interleukin-7 (IL-7), interleukin-8 (IL-8), interleukin-11 (IL-11), interleukin -12 (IL-12), leukemia inhibitory factor (LIF), and tumor necrosis factor-alpha (TNF-α).

To the SC CM fraction also other substances may be added including for instance Ca++ ions which can activate the platelets of the PRP substances..

Preferably, in the composition according to the present invention the stem cells from which the SC CM is obtained are isolated from bone marrow or adipose tissue.

In particular, the composition according to the present invention further comprises DMSO. Dimethyl sulfoxide (DMSO) is an organosulfuric compound with the formula (CH₃)₂SO. DMSO is a colourless liquid and an important polar aprotic solvent that dissolves both polar and nonpolar compounds, miscible in a wide range of organic solvents as well as water. The addition of DMSO to the composition according to the invention was found to be particularly helpful and additionally improved the effectivity of the composition for the treatment of tissue injuries in a subject, and in particular for wound healing purposes.

By using the composition comprising PRP and SC CM combined with DMSO a very efficient wound healing process has been observed. The wound closure time is much shortened even further and the healing is better (granulation, pink colour, closure time). The different phases inflammation phase, proliferation phase and maturation phase are shortened.

Preferably DMSO is present in the composition according to the present invention in amount ranging between 5% and 25%, preferably between 7.5% and 20% and more preferably about 10%, 15% or 20%.

Also, while the composition according to the present invention may be used in liquid form (in a treatment bath or as a spray), the effectivity may be further improved by providing the composition according to the invention in the form of a gel. Accordingly, the composition according to the invention preferably further comprises a gelling agent. Accordingly, the composition according to the invention can be applied as a topical treatment.

In preferred embodiments, the ratio in which said PRP and SC CM are present in the composition according to the invention range between about 3:1 to about 1:3, preferably between about 2:1 to about 1:2, and more preferably 2:1, 1:1 or 1:2.

In a particular embodiment, in the composition according to the present invention conditioned medium is a processed conditioned medium. The term "processed conditioned medium" refers to a conditioned medium that has undergone several treatments such as washing, purification and/or feeding steps. The medium preferably harvested after a confluent growth of the cell culture.

In a second aspect, the present invention relates to a composition according to the above for use in medicine, preferably animal medicine.

In particular the composition according to the invention has been shown to have an increased and synergetic clinical effect in the regeneration of the tissue (wound healing). It has been found that the composition promotes tissue epithelialisation and granulation of wounds. It can be applied on superficial and deep wounds as a result of a trauma, surgery or any other external tissue damage where skin closure is a problem. The compositions according to the invention have been found to heal wounds more rapidly compared to conventional treatments and the compositions according to the invention can therefore be used to accelerate the healing process and reduce scar formation.

Preferably, the present invention relates to a composition according to the above for use in the treatment of skin wounds in a subject.

It has also been found that both the PRP and the SC CM as present in the compositions according to the invention may be from either "autogenic" donors or "allogeneic" donors. This simplifies the treatment as it is no longer necessary that the donor of the source of the compositions according to the invention, the donor of the stem cells or the blood, is to be the same as the receiver of the treatment. Autogenic methods which typically use the patient's own blood and stem cells are more expensive as they require an individualized approach, while for an allogeneic method blood and/or stem cells donated by one or more third parties can be used for preparing the compositions. This allows the production of larger quantities, hence reducing the production costs drastically.

Accordingly, the present invention relates to a composition according to the above for use in the allogeneic treatment of skin wounds in a subject. Preferably said subject is a mammal, preferably selected from domestic animals such as dogs, cats and rabbits or farm animals such as horses, cattle, sheep and goats.

The compositions according to the invention are applied on a cleaned wound. The composition, preferably in the form of a gel, can be applied on the wound directly or using a sterile dressing. Typically only a single application on the wound is sufficient, but depending on the size and the appearance of the wound treatment can be repeated in a week interval.

In a further aspect, the present invention relates to a composition according to the above for use in the treatment of mastitis in a subject.

The compositions according to the invention has been shown to reduce the inflammation of the mammary glands and to promote the healing of wounds which can occur in the nipple tissue during mastitis.

As described above, it has also been found that both the PRP and the SC CM as present in the compositions according to the invention may be from either "autogenic" donors or "allogeneic" donors.

Accordingly, the present invention relates to a composition according to the above for use in the allogeneic treatment of skin wounds in a subject. Described is the use of a composition according to the above, for promoting hair growth as an essential part of skin regeneration in a mammalian subject.

Additionally, it has been observed that the compositions as described above are shown to promote hair growth in mammals. Disclosed is the use of the compositions to promote hair growth in mammals wherein the hair is selected from those on the top of the head, on the armpits, on the pubic area, on the face including eyelash, eyebrow, eyelid, moustache, beard and whisker, on the chest, arms and legs, preferably hair is selected from those on scalp, beard, head, pubic area, upper lip, eyelash, eyebrow, and eyelid.

In a further aspect, the present invention relates to a method for preparing a composition according to the invention, comprising at least the blending of platelet enriched plasma (PRP) and stem cell conditioned medium (SC CM), wherein said SC CM is the medium harvested after culturing of mesenchymal stem cells. The process includes the collection of the secretes of mesenchymal stem cells that activates the gelling of the platelet enriched plasma. The formation of a glue appears after about 6 hours.

The present invention is hereafter exemplified by the illustration of particular, nonlimiting examples.

### EXAMPLES

### EXAMPLE 1 - Wound healing study

A study was conducted using the composition according to the invention. The composition consisted of 30 % of platelet enriched plasma, 60% of Mesenchymal Stem Cell Conditioned Medium and 10 % of DMSO.

Hedgehogs which were diagnosed with skin wounds were treated with the treatment composition comprising platelet enriched plasma and Mesenchymal Stem Cell Conditioned medium. An overview of the treatment outcome is given in table 1.

**Table 1**

| Subject | Wound description | Treatment (days) | | | | | Result |
|---|---|---|---|---|---|---|---|
| | | 0 | 2 | 5 | 7 | 8 | |
| 1 | Small wound on right elbow | A | + | +++ | - | RA | Healed |
| 2 | Long lasting wound on face | A | + | +++ | +++ | RA | Healed |
| 3 | Infection of the eye orbit | A | + | +++ | +++ | RA | Healed |
| 4 | Skin infection and sarcoptes | A | / | +++ | + | | Healed |
| 5 | Deep wound on jaw | A | - | - | + | | Improved |

| | | | | | | | |
|---|---|---|---|---|---|---|---|
| A = Application of the treatment composition RA = Reapplication of the treatment composition - = No further improvement + = Minor improvement +++ = Great improvement | | | | | | | |

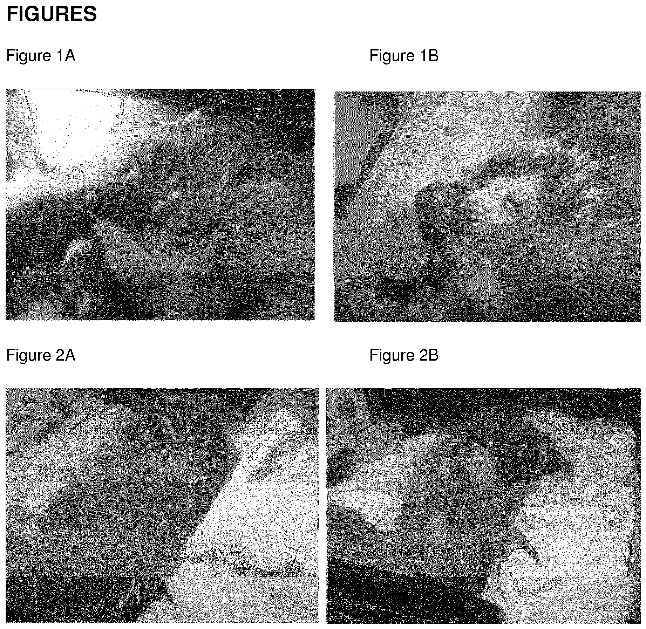

Figure 1 A and B show the improvement over time of the wound of subject 3

Figure 2 A and B show the improvement over time of the wound of subject 4

### EXAMPLE 2 - Hair growth study

Hedgehogs which were at least partially bold because of scabies were treated with the treatment composition comprising platelet enriched plasma and Mesenchymal Stem Cell Conditioned medium. The boldness was caused by damage to the roots of the spines.

The composition as used in example 1 was applied and after 10 days it was observed that new spines appeared.

### EXAMPLE 3 - Wound healing study

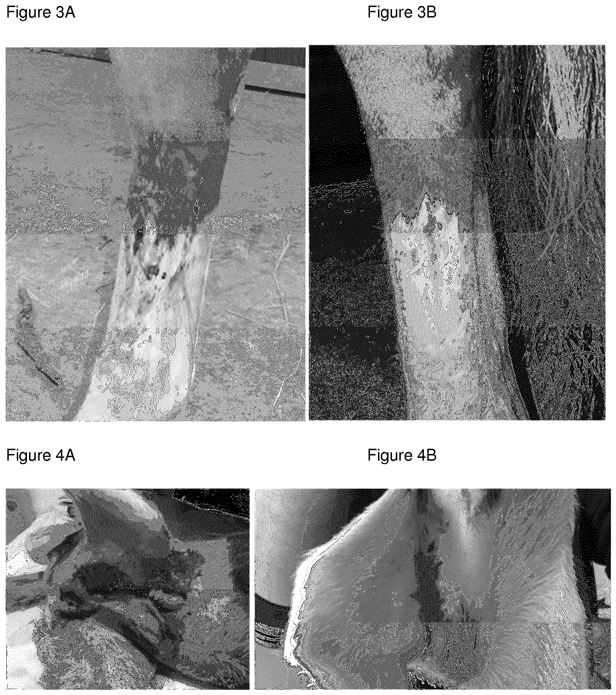

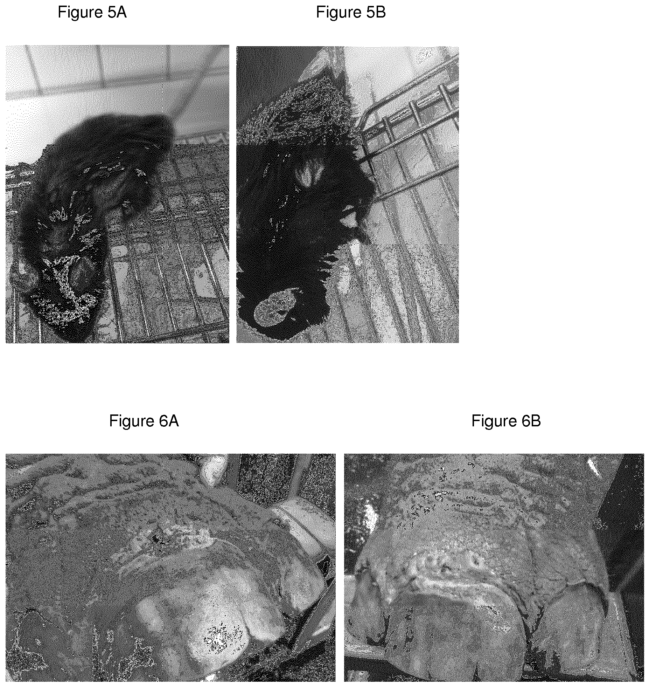

The composition as used in example 1 was also successfully used to treat wounds in horses, dogs, mice suffering from ulcerative dermatitis and elephants affected by hoof lesions. The composition as used in example 1 was applied to severe wounds in horses (figure 3A), dogs (figure 4A), mice (figure 5A) and elephants (figure 6A) and after about two weeks the wounds were almost completely healed (figures 3B, 4B, 5B and 6B).

### EXAMPLE 4 - Wound healing study

### Experimental Animals

All animal experiments were approved by the Animal Ethics Committee. Sixty 8-week old (22-26 grams) male/female mice were kept in standard conditions of 21 °C and a normal light-dark cycle with free access to food and water.

### Anesthesia and operative preparation

General anesthesia was induced using 5% isoflurane in 100% oxygen (flow rate 1 L/min) and maintained using 1-3% isoflurane. The suppression of deep pedal reflexes of the mouse was ensured and the mouse was placed in the prone position. Next, the operative region was prepared by removing to 3cm round the place of cutting between the two shoulder blades of the mouse. Subsequently, the skin was wiped with an alcohol swab and two applications of 10% povidone-iodine (Betadine) and draped.

### Excision

A scalpel was used to make a 1 cm longitudinal injury on either side of the mouse's midline at the level of the shoulders. Next, a serrated forceps was used to lift the skin in the middle of the outline and iris scissors to create a full-thickness wound that extends through the subcutaneous tissue. This process was repeated for the wound on the other side of the midline. The therapeutic compound was applied to one wound (right side) and the control to the other (left side) (see Table 2). Following topical applications were applied: growth factors from blood (product A), growth factors from adipose stem cells (product B), or both (product A+B in gel) (see Table 2). Finally, the wound was not sutured and no special second dressing was used.

### Postoperative Management

Carprofen (5 mg/kg) was administered once daily via sub-cutaneous injection for postoperative pain relief. After the surgery animals were caged individually and maintained until fully recovered. The animals were monitored twice daily for manifestations of pain and weight loss. No gross behavioral displays of pain or weight loss were observed.

**Table 2**

| Group U.D. | 20 mice with U.D. Treated with AB gel | | 20 mice with U.D Not treated | |
|---|---|---|---|---|
| Side of wound | Left | Right | Left | Right |
| Group 1 (=10 + 10 mice) | Vehicle | A | A | B |
| Group 2 (=10 + 10 mice) | Vehicle | B | B | A |
| Group 3 (=10 + 10 mice) | A | AB | B | AB |

### Wound Measurement and Treatment

The wound was measured daily. General anesthesia was induced using 5% isoflurane gas (flow rate 1 L/min), and subsequently the suppression of deep sensory reflexes of the mouse was ensured using 1-3% isoflurane. Next, the occlusive dressing was peeled back gently with forceps. Surgical calipers were used to measure the wound diameter. The average of three measurements was taken along the X, Y and Z. When considered necessary, the therapeutic compound and the vehicle control were re-applied at this point. Finally, a clean transparent occlusive dressing was re-applied and the animals were kept warm until fully recovered. At the completion of experiments, wound closure, morphological architecture and degree of neovascularization, granulation were observed and scored.

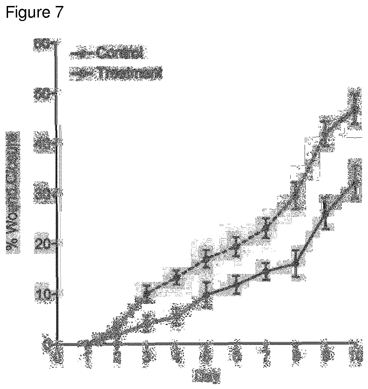

A wound closure curve was determined by calculating the average diameter of the wound and expressing the results as a percentage, *i.e.* 100 - (Day 0 diameter/Day X diameter). In this experiment a therapeutic compound (or vehicle control) was applied daily to the wound. The therapeutic compound greatly accelerated wound closure (Figure 7).

## Claims

1. Composition comprising platelet enriched plasma (PRP) and stem cell conditioned medium (SC CM) and optionally DMSO and/or a gelling agent, wherein said SC CM is the medium harvested after the culturing of mesenchymal stem cells.

2. Composition according to claim 1, wherein said PRP comprises transforming growth factor-β (TGF-β), fibrinogen, platelet-derived growth factor (PDGF), epidermal growth factor (EGF), transforming growth factor-α (TGF-α), vascular endothelial growth factor (VEGF), platelet thrombo-plastin, thrombospondin, coagulation factors, calcium, serotonin, histamine, and hydrolytic enzymes.

3. Composition according to claim 1 or 2, wherein said SC CM comprises hepatocyte growth factor, transforming growth factor β (TGF-β), anti -apoptopic factors, keratinocyt growth factor, brain-derived neurotrophic factor (BDNF), Flt-3 ligand, granulocyte colony stimulating factor (G-CSF), granulocyte/macrophage colony stimulating factor (GM-CSF), macrophage colony stimulating factor (M-CSF), interleukin-6 (IL-6), interleukin-7 (IL-7), interleukin-8 (IL-8), interleukin-11 (IL-11), interleukin leukin-12 (IL-12), leukemia inhibitory factor (LIF), and tumor necrosis factor-alpha (TNF-α).

4. Composition according to any of claims 1 to 3, wherein said PRP and SC CM are present in the composition in a ratio of about 3:1 to about 1:3.

5. Composition according to any of claims 1 to 4, wherein the stem cells are isolated from bone marrow or adipose tissue.

6. Composition according to any of claims 1 to 5, wherein said conditioned medium is a processed conditioned medium.

7. Composition according to any of claims 1 to 6, for use in medicine.

8. The composition according to claim 1 to 6, for use in the treatment of mastitis in a subject.

9. The composition according to claim 1 to 6, for use in the treatment of skin wounds in a subject.

10. The composition for use according to claim 8 or 9, wherein said subject is a mammal, preferably selected from domestic animals such as dogs, cats and rabbits or farm animals such as horses, cattle, sheep and goats.

11. Method for preparing a composition according to any of claims 1 to 6, comprising at least the blending of platelet enriched plasma (PRP) and stem cell conditioned medium (SC CM), wherein said SC CM is the medium harvested after the culturing of mesenchymal stem cells.

## Patentansprüche

1. Zusammensetzung, umfassend mit Thrombozyten angereichertes Plasma (Platelet Enriched Plasma, PRP) und mit Stammzellen konditioniertes Medium (Stem Cell Conditioned Medium, SC CM), und gegebenenfalls DMSO und/oder ein Geliermittel, wobei es sich bei dem SC CM um das Medium handelt, das nach dem Kultivieren mesenchymaler Stammzellen geerntet wird.

2. Zusammensetzung nach Anspruch 1, wobei das PRP TGF-β (Transforming Growth Factor-β), Fibrinogen, PDGF (Platelet-Derived Growth Factor), EGF (Epidermal Growth Factor), TGF-α (Transforming Growth Factor-α), VEGF (Vascular Endothelial Growth Factor), Thrombozyten-Thromboplastin, Thrombospondin, Gerinnungsfaktoren, Calcium, Serotonin, Histamin und hydrolytische Enzyme umfasst.

3. Zusammensetzung nach Anspruch 1 oder 2, wobei das SC CM Hepatozyten-Wachstumsfaktor, TGF-β (Transforming Growth Factor-β), antiapoptopische Faktoren, Keratinozyten-Wachstumsfaktor, BDNF (Brain-Derived Neurotrophic Factor), Flt-3-Ligand, G-CSF (Granulocyte Colony Stimulating Factor), GM-CSF (Granulocyte/Macrophage Colony Stimulating Factor), M-CSF (Macrophage Colony Stimulating Factor), Interleukin-6 (IL-6), Interleukin-7 (IL-7), Interleukin-8 (IL-8), Interleukin-11 (IL-11), Interleukin Leukin-12 (IL-12), LIF (Leukemia Inhibitory Factor) und TNF-α (Tumor Necrosis Factor-alpha) umfasst.

4. Zusammensetzung nach einem der Ansprüche 1 bis 3, wobei das PRP und das SC CM in der Zusammensetzung in einem Verhältnis von etwa 3:1 bis etwa 1:3 vorliegen.

5. Zusammensetzung nach einem der Ansprüche 1 bis 4, wobei die Stammzellen aus Knochenmark oder Fettgewebe isoliert werden.

6. Zusammensetzung nach einem der Ansprüche 1 bis 5, wobei es sich bei dem konditionierten Medium um ein prozessiertes konditioniertes Medium handelt.

7. Zusammensetzung nach einem der Ansprüche 1 bis 6, zur medizinischen Verwendung.

8. Zusammensetzung nach Anspruch 1 bis 6, zur Verwendung bei der Behandlung von Mastitis bei einem Individuum.

9. Zusammensetzung nach Anspruch 1 bis 6, zur Verwendung bei der Behandlung von Hautwunden bei einem Individuum.

10. Zusammensetzung nach Anspruch 8 oder 9, wobei es sich bei dem Individuum um einen Säuger, vorzugsweise ausgewählt aus Haustieren wie Hunden, Katzen und Kaninchen oder Nutztieren wie Pferden, Rindern, Schafen und Ziegen, handelt.

11. Verfahren zur Herstellung einer Zusammensetzung nach einem der Ansprüche 1 bis 6, umfassend wenigstens das Mischen von mit Thrombozyten angereichertem Plasma (PRP) und mit Stammzellen konditioniertem Medium (SC CM), wobei es sich bei dem SC CM um das Medium handelt, das nach dem Kultivieren mesenchymaler Stammzellen geerntet wird.

## Revendications

1. Composition comprenant du plasma enrichi en plaquettes (PRP) et un milieu conditionné pour cellules souches (SC CM) et, en option, du DMSO et/ou un agent gélifiant, dans laquelle ledit SC CM est le milieu recueilli après la culture de cellules souches mésenchymateuses.

2. Composition selon la revendication 1, dans laquelle ledit PRP comprend le facteur de croissance transformant β (TGF-β), le fibrinogène, le facteur de croissance dérivé des plaquettes (PDGF), le facteur de croissance épidermique (EGF), le facteur de croissance transformant α (TGF-α), le facteur de croissance de l'endothélium vasculaire (VEGF), la thromboplastine des plaquettes, la thrombospondine, des facteurs de coagulation, le calcium, la sérotonine, l'histamine et des enzymes hydrolytiques.

3. Composition selon les revendications 1 ou 2, dans laquelle ledit SC CM comprend le facteur de croissance des hépatocytes, le facteur de croissance transformant β (TGF-β), des facteurs anti-apoptotiques, le facteur de croissance des kératinocytes, le facteur neurotrophique issu du cerveau (BDNF), un ligand Flt-3, le facteur stimulant les colonies de granulocytes (G-CSF), le facteur stimulant les colonies de granulocytes/macrophages (GM-CSF), le facteur stimulant les colonies de macrophages (M-CSF), l'interleukine-6 (IL-6), l'interleukine-7 (IL-7), l'interleukine-8 (IL-8), l'interleukine-11 (IL-11), l'interleukine-12 (IL-12), le facteur inhibiteur de leucémie (LIF) et le facteur de nécrose tumorale alpha (TNF-α).

4. Composition selon l'une quelconque des revendications 1 à 3, dans laquelle lesdits PRP et SC CM sont présents dans la composition en un rapport de 3:1 environ jusqu'à 1:3 environ.

5. Composition selon l'une quelconque des revendications 1 à 4, dans laquelle les cellules souches sont isolées à partir de moelle osseuse ou de tissu adipeux.

6. Composition selon l'une quelconque des revendications 1 à 5, dans laquelle ledit milieu conditionné est un milieu conditionné traité.

7. Composition, selon l'une quelconque des revendications 1 à 6, destinée à être utilisée en médecine.

8. Composition, selon l'une quelconque des revendications 1 à 6, destinée à être utilisée dans le traitement d'une mastite chez un sujet.

9. Composition, selon l'une quelconque des revendications 1 à 6, destinée à être utilisée dans le traitement de plaies cutanées chez un sujet.

10. Composition destinée à être utilisée selon les revendications 8 ou 9, dans laquelle ledit sujet est un mammifère, de préférence choisi parmi des animaux domestiques, tels que des chiens, chats et lapins, ou des animaux de la ferme, tels que des chevaux, bétail, moutons et chèvres.

11. Procédé destiné à préparer une composition selon l'une quelconque des revendications 1 à 6, comprenant au moins le mélange de plasma enrichi en plaquettes (PRP) et d'un milieu conditionné pour cellules souches (SC CM), dans lequel ledit SC CM est le milieu recueilli après la culture de cellules souches mésenchymateuses.
